Europäisches Patentamt

**European Patent Office**

**Office européen des brevets**

(11) Publication number: **0 187 708 B1**

## EUROPEAN PATENT SPECIFICATION

(45) Date of publication of patent specification: **12.08.92** (51) Int. Cl.5: **B01D 63/02, A61M 1/18**

(21) Application number: **86300074.1**

(22) Date of filing: **07.01.86**

(54) Mass transfer device having a microporous, spirally wound hollow fiber membrane.

(30) Priority: **08.01.85 US 689613**
**12.12.85 US 806378**

(43) Date of publication of application:
**16.07.86 Bulletin 86/29**

(45) Publication of the grant of the patent:
**12.08.92 Bulletin 92/33**

(84) Designated Contracting States:
**BE DE FR GB IT NL**

(56) References cited:
**EP-A- 0 053 635**    **EP-A- 0 089 122**
**EP-A- 0 103 899**    **EP-A- 0 114 732**
**FR-A- 2 325 408**    **FR-A- 2 367 519**
**FR-A- 2 381 000**    **FR-A- 2 542 203**
**GB-A- 2 012 187**    **US-A- 3 422 008**
**US-A- 4 368 124**

(73) Proprietor: **MEDTRONIC, INC.**
**7000 Central Avenue N.E.**
**Minneapolis, Minnesota 55432-3576(US)**

(72) Inventor: **Badolato, Anthony**
**38 Eden Rock Lane**
**Willingboro, N.J. 08046(US)**
Inventor: **Corey, Edmund R., Jr.**
**The Meadows 1602 Meadow View Lane**
**Montclare, PA 19453(US)**
Inventor: **Barrera, James G.**
**18 Brinfield Road**
**Audubon, PA 19403(US)**

(74) Representative: **Jones, Alan John et al**
**CARPMAELS & RANSFORD 43 Bloomsbury Square**
**London, WC1A 2RA(GB)**

## Description

### Field of the Invention

The present invention relates to an axial flow, spirally wound hollow fiber blood oxygenator and to a method for extracorporeally oxygenating the blood of an animal or human.

### Background of the Invention

Blood oxygenator systems have been used for some time now in open heart surgery and for providing emergency cardiopulmonary assistance. In both instances, the oxygenator takes over, either partially or completely, the normal gas exchange function of the patient's lung. In oxygenators which employ a microporous membrane, blood is taken from the patient and is circulated extracorporeally through the oxygenator on one side of the membrane. Concurrently, an oxygenating gas is passed through the oxygenator on the other side of the membrane. Carbon dioxide is transferred from the blood across the microporous membrane into the passing stream of oxygenating gas; at the same time, oxygen is transferred from the oxygenating gas across the membrane into the blood. The circulating blood, having thereby been reduced in carbon dioxide content and enriched in oxygen, is returned to the patient. Blood is circulated, oxygenated and returned to the patient in the aforementioned manner until the patient's own cardiopulmonary system is once more able to carry out its normal circulatory and gas exchange functions.

Several types of blood oxygenators have been or are generally available. One type is a bubble oxygenator wherein the oxygenating gas is introduced into the blood directly in the form of bubbles. In a second type of oxygenator, called a film-type oxygenator, a thin blood film is made and gas exchange takes place on the surface of the exposed blood film. A third type of blood oxygenator is called a membrane oxygenator. In the membrane oxygenator, the blood is separated from direct contact with the oxygenating gas by a membrane. This membrane must be microporous or semipermeable, that is, the membrane must be capable of permitting carbon dioxide and oxygen to permeate through it while at the same time preventing the blood itself from passing therethrough.

There are two types of membrane blood oxygenators currently available. One type, called the flat plate membrane oxygenator, employs one or more thin, flat sheets of microporous membrane. In its most basic form the flat plate oxygenator has a single sheet of microporous membrane sealed into a housing so as to provide in the housing a first compartment (the "blood compartment") for the flow of blood and a second compartment (the "gas compartment") for the flow of an oxygenating gas. Each of the compartments is fitted with an inlet and an outlet. Blood flows into and out of the blood compartment and the oxygenating gas flows into and out of the gas compartment. Oxygen passes from the oxygenating gas across the membrane into the blood flowing through the blood compartment.

Carbon dioxide passes from the entering blood across the membrane to be entrained in the oxygenating gas. The exiting blood, now reduced in carbon dioxide and enriched in oxygen, is returned to the patient.

The other type of membrane oxygenator, referred to as a hollow fiber oxygenator, is illustrated generally in U.S. 4,239,729 to Hasegawa et al. A hollow fiber oxygenator employs a large plurality (typically, thousands) of microporous or semipermeable hollow fibers disposed within a housing. These hollow fibers are sealed in the end walls of the housing which are then fitted with skirted end caps. One end cap is fitted with an inlet, the other end cap is fitted with an outlet. The peripheral wall of the housing has an inlet located interiorly of one of the end walls and an outlet located interiorly of the other end wall. In the Hasegawa et al. oxygenator, the hollow fibers are aligned in the housing so that their longitudinal axes are generally parallel to the longitudinal axis of the housing. In the Hasegawa et al. device, blood enters through the inlet of one end cap, passes through the lumens of the hollow fibers, and exits through the outlet of the other end cap. The oxygenating gas enters the device through the inlet in the peripheral wall near one end of the device, passes over the outer surfaces of the hollow fibers, and exits the device through the outlet in the peripheral wall near the other end of the device. It will be understood that carbon dioxide diffuses from the blood flowing inside the hollow fibers through the fiber walls into the stream of oxygenating gas. At the same time, oxygen from the oxygenating gas flowing over the outer surfaces of the hollow fibers diffuses through the walls of the hollow fibers into the lumens thereof to oxygenate the blood flowing therethrough.

A hollow fiber oxygenator with an integral heat exchanger has recently become available from the Terumo Corporation under the designation Capiox II. The hollow fibers in the Terumo oxygenator are arranged as the hollow fibers in the Hasegawa et al. device, i.e. the longitudinal axes of the fibers are parallel to the longitudinal axis of the oxygenator housing. In use, blood is passed through the lumens of the hollow fibers while the oxygenating gas is passed over the outer peripheral surfaces of the fibers.

The Terumo oxygenator uses hollow fibers whose inside diameters are about 200 microns and whose wall thickness is about 25 microns. The effective length of the hollow fibers (i.e. the straight line distance between the innermost surfaces of the end walls in which the fibers are imbedded) is about 130-140 mm. depending on the size of the oxygenator. The manufacturer supplies the device in several sizes as measured by the surface area to which the blood to be oxygenated is exposed. Where the blood passes through the lumens of the hollow fibers, as is the case in the Terumo or Hasegawa et al. devices, the surface area, S.A., to which the oxygenating gas is exposed is given by equation (1):

$$(1) \quad S.A. = \pi(I.D.)(length)(n)$$

where (I.D.) is the inside diameter of the fibers, (length) is the effective length of the fibers in the device and (n) is the total number of fibers.

The Terumo oxygenator is currently provided by the manufacturer in four different surface areas, i.e., 1.6 $m^2$, 3.3 $m^2$, 4.3 $m^2$, and 5.4 $m^2$, the largest of these sizes being intended for use on adult patients and the smallest being intended for use on infants. Using these surface areas and the aforementioned inside diameter of the fibers in equation (1), one can calculate that the Terumo oxygenator employs about 62,000, about 53,000, about 20,000 and about 18,000 fibers, respectively, to provide gas exchange surface areas of approximately 5.4 $m^2$, 4.3 $m^2$, 3.3 $m^2$, and 1.6 $m^2$, respectively. Thus, it is seen that even the smallest Terumo oxygenator uses a very large number (about 20,000) of microporous fibers. This large number of fibers makes the oxygenator difficult to assemble and, since the fibers are expensive, adds to the selling price of the final product. Since the Terumo device is designed to have blood flow through the lumens of its hollow fibers, and since those hollow fibers have relatively small inside diameters, there are relatively large blood pressure drops associated with the device. For example, at a surface area of 5.4 $m^2$ and a blood flow of 6 liters per minute, the blood pressure drop is said to be 175 mm. Hg. As another example, at a surface area of 1.6 $m^2$ and a blood flow of 2 liters per minute, the blood pressure drop is said to be about 150 mm. Hg.

US-A-3422008 to McLain discloses a permeability separatory apparatus comprising selectively permeable hollow fibers which are wound spirally around a cylindrical core through a substantial portion of the length of the core. In one embodiment of the separatory apparatus, a region near each end of the core is impregnated with a casting resin so as to form a flange extending annularly and perpendicularly from the core. These flanges are subsequently cut perpendicularly to the axis of the core so as to provide open ends in the fibers at the outer surface of each flange. The core/flange combination is then placed into a generally cylindrical casing and the outer peripheral surface of each flange is sealed in fluid tight relationship to the adjacent inner surface of the casing.

McLain employs one or more continuous hollow fibers and winds the fiber spirally on a cylindrical supporting core. The preferred method of wrapping the fiber on the supporting core consists of revolving the core on its linear axis and then feeding one or more continuous hollow fibers so that the fiber is wound around the core as the core is rotated. A guide positions the fiber on the core as the guide traverses the length of the core, the guide changing direction as it reaches each respective end of the core.

A spirally wound hollow fiber oxygenator device has recently been brought to market by C. R. Bard. Upon examination of this device, it appears that a "ribbon" of about 100 or so continuous hollow fibers has been spirally wrapped on a support core and it appears also that the winding process was carried out using the McLain apparatus and procedure just discussed.

The U.S. patent directly corresponding to the Bard device is believed to be US-A-4424190. This U.S. patent describes a hollow fibre blood oxygenator in which the blood flows round the exterior of the hollow fibres, the oxygenating gas being passed through the fibre lumens. The fibres are arranged as a mat of a plurality of fibre layers, which are disposed between a porous core and an outer cylindrical wall. The ends of the fibres are sealed in resin tubesheets, which also seal fibres to the porous core and the outer cylindrical wall. In use, oxygenating gas passes along the fibre lumens, whilst blood is passed into the hollow centre of the core and thence flows radially outwardly through the porous wall of the cores. The blood thus flows radially through the fibre mat where it is oxygenated, into an annular space between the fibre mat and the outer wall, whence it returns through an outlet to the patient.

EP-A-89122 discloses a device which functions in precisely the manner described above with reference to US-A-4424190.

According to the invention, there is provided a spirally wound hollow fibre oxygenator, comprising: one or more fibre ribbons, each including one or more gas permeable fibres having continuous lumens therethrough, wound spirally around a core along a substantial portion of the length of the core; a housing within which the one or more fibres and the core are disposed such that the one or more fibres are contained in an annular chamber whose peripheral walls are formed by the housing and the core in such a way that a first plurality of fibre ribbons are wound in one sense, and a second plurality of fibre ribbons are wound in the other sense; an annulus of casting resin located between and sealed to the core and the housing at each end of the core, the end portions of the one or more fibres being sealed in the resin annuli and the ends of the one or more fibres being open at the outer end surfaces of the resin annuli, and the resin annuli defining the ends of the chamber containing the one or more fibres; end caps fitted to the housing, one of which end caps has an inlet port for admission of oxygenating gas to the lumens of the fibres and the other of which has an outlet port for exit of oxygenating gas from the lumens of the fibres; an inlet for admission of blood to the annular chamber containing the one or more fibres; and an outlet for exit of blood from the annular chamber containing the one or more fibres; characterised in that: the blood inlet and the blood outlet are arranged in the peripheral walls of the annular chamber and spaced axially from one another at opposite ends of the chamber; and the fibre ribbon windings occupy substantially all of the annular chamber, thereby constraining the blood to flow axially through the fibre ribbon windings.

The oxygenator may be provided with an integral heat exchanger. Thus, for example, the temperature of the blood may be raised or lowered as desired during a surgical procedure.

Blood oxygenators made in accordance with the teachings of the present invention employ much less hollow fibre surface area than prior art devices as a result of which there is less foreign body surface area (i.e. the surfaces of the hollow fibres) which the blood may contact.

Despite the fact that there is less hollow fiber surface area compared to prior art devices, the mass transfer efficiency of an oxygenator made in accordance with the present invention is equal to or better than known prior art hollow fiber oxygenators The oxygenator of the present invention has a greatly reduced priming volume and is much more compact than existing devices.

## BRIEF DESCRIPTION OF THE DRAWINGS

FIG. 1 is a perspective of a spirally wound hollow fiber oxygenator in accordance with the present invention;
FIG. 2 is a top plan view of the oxygenator of FIG. 1;
FIG. 3 is a vertical cross-section of the oxygenator of FIG. 1;
FIG. 4 is an exploded view of the oxygenator of FIG. 1 and showing the top header, the outer casing, inner support core, and the bottom header with optional heat exchanger;

FIG. 5 is a bottom plan view, showing the inner surfaces, of the top header of the oxygenator;
FIG. 6 is a section taken along line 6-6 of FIG. 5;
FIG. 7 is a top perspective of the outer casing of the oxygenator;
FIG. 8 is a "front" perspective of the outer casing as viewed perpendicularly to line 8-8 of FIG. 7;
FIG. 9 is a "side" perspective of the outer casing viewed perpendicularly to line 9-9 of FIG. 8;
FIG. 10 is a perspective, with parts in cross-section, of the inner core and core extenders prior to start of the fiber winding operation;
FIG. 11 is a highly magnified view of a ribbon of six hollow fibers and their spacing as they are about to be wound on the inner support core;
FIG. 12 is a view of the support core with its core extenders in place showing the location of a ribbon of six hollow fibers on the core at an early stage of the fiber winding process;
FIG. 13 is a greatly enlarged view of the dot-and-dashed portion of FIG. 12;

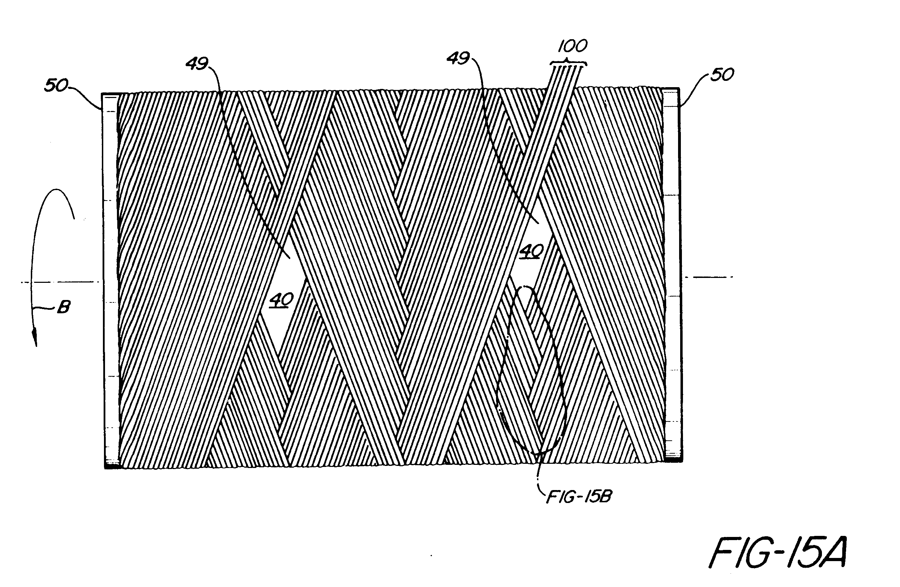

FIG. 14 is a sectional view taken along line 14-14 of FIG. 13;
FIG. 15A is a view similar to FIG. 12 showing the placement of fibers on the support core at a later stage of the winding process;
FIG. 15B is a greatly magnified view of the circled portion of FIG. 15A;
FIG. 15C is a cross-section taken along line 15C-15C of FIG. 15B;
FIG. 16A is a view similar to that of FIGS. 12 and 15A showing the placement of fibers at a still later stage of the winding process;
FIG. 17 is a very greatly enlarged view showing the path of a first fluid through the interiors of the hollow fibers and the highly random path which a second fluid might take as it flows through the fiber bundle during operation of the device; and
FIG. 18 is a sectional view, with parts cut away and other parts in phanton, showing the inner core (with core extenders at each end and fibers wrapped around the extended core) disposed in an outer casing at an intermediate stage of the end wall potting operation.

## Detailed Description of the Invention

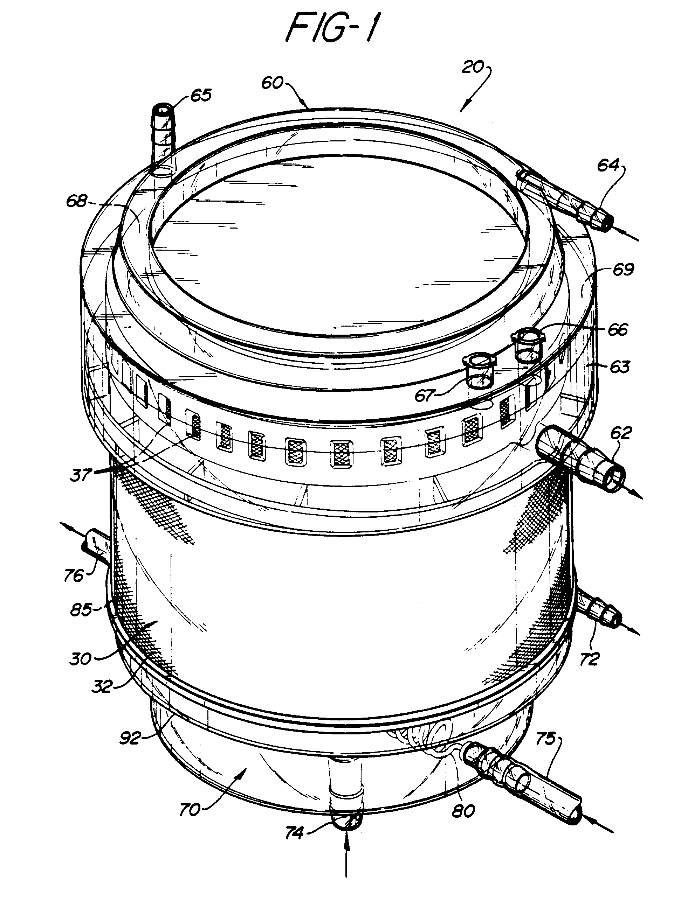

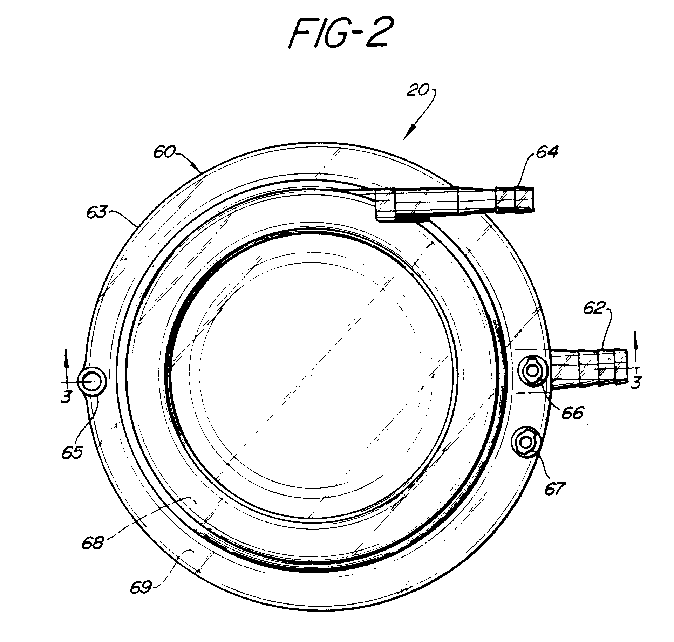

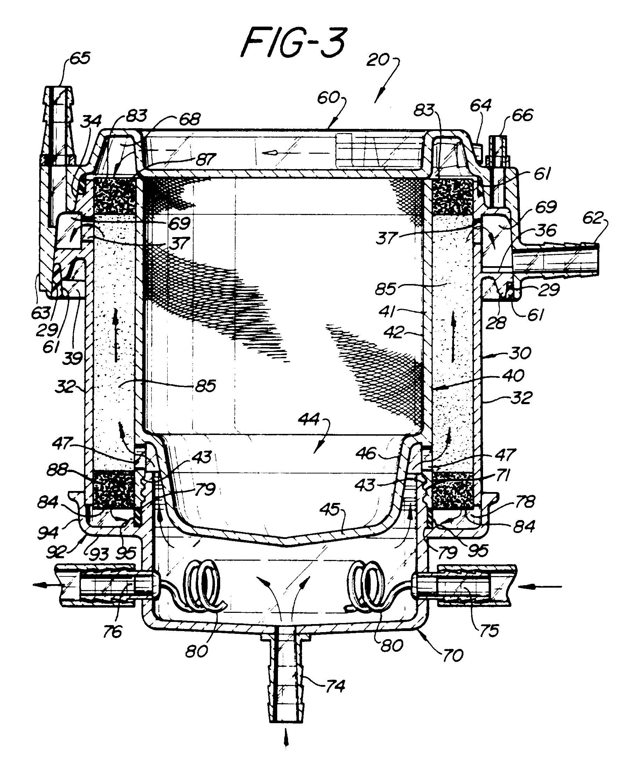

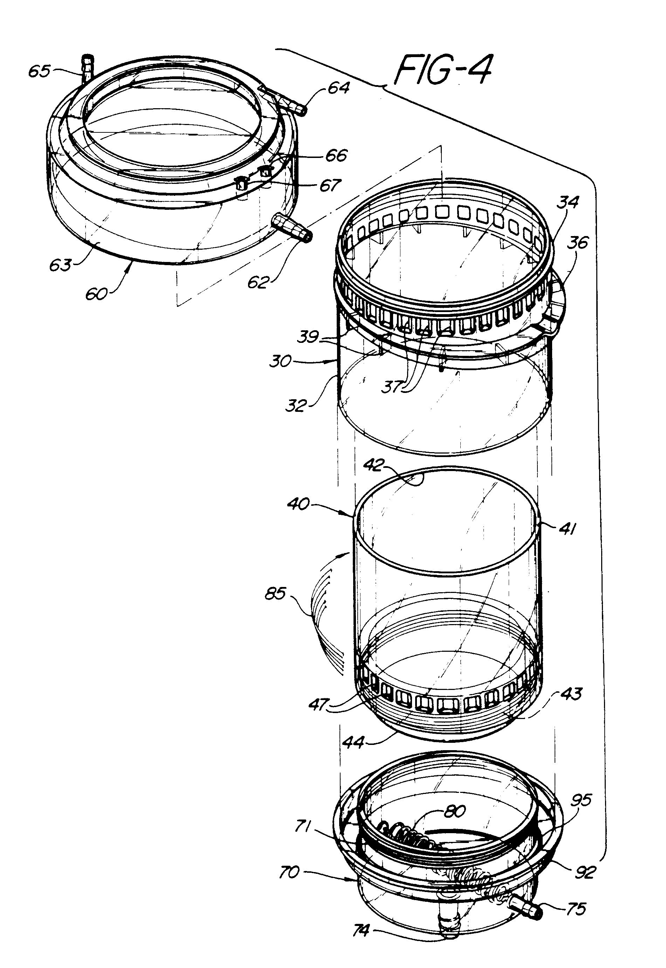

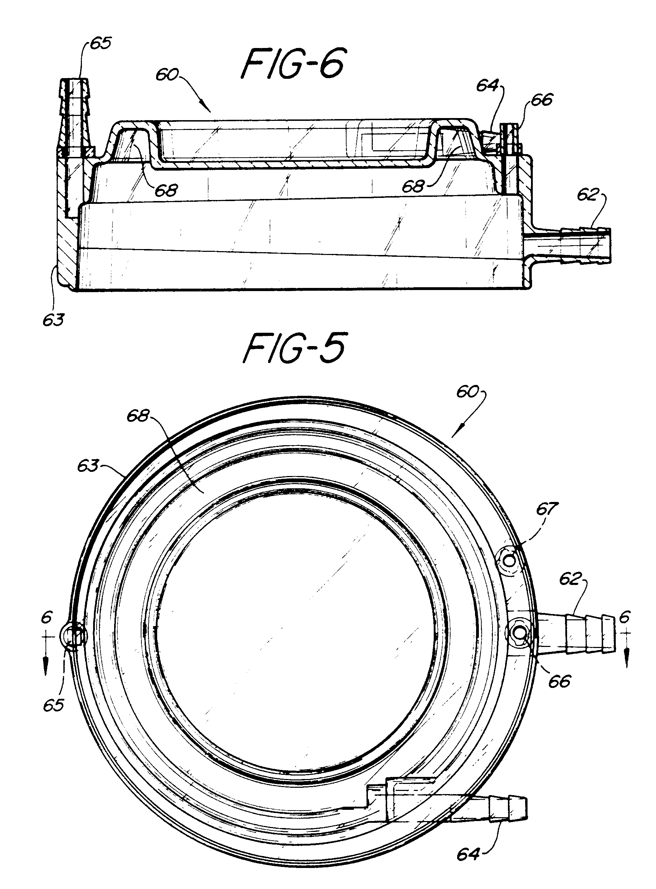

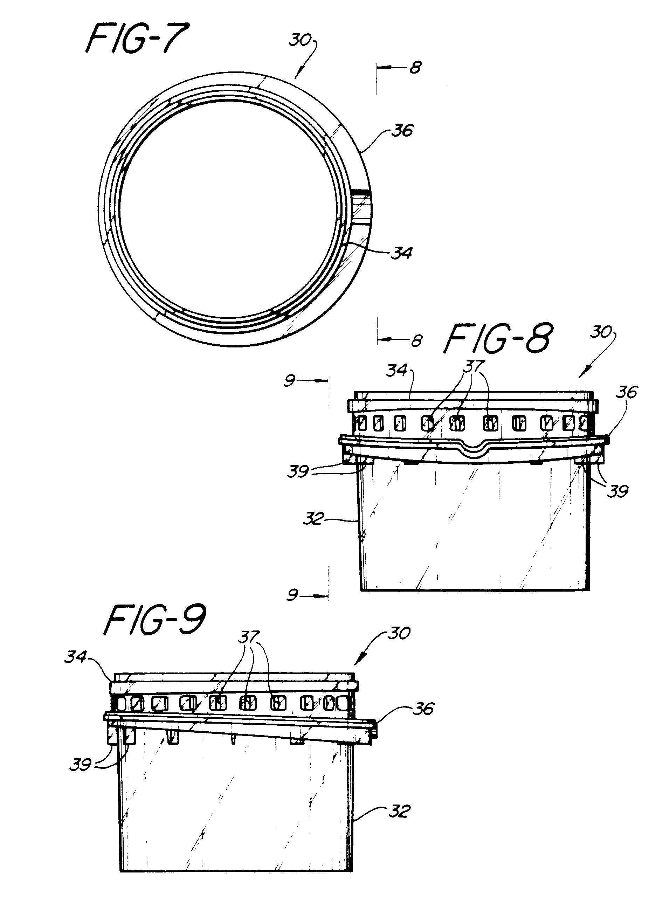

Referring now to the drawings, and especially to Figs. 1-10 thereof, a blood oxygenator 20 in accordance with the present invention comprises a cylindrical outer casing 30, a cylindrical inner support core 40 on which are wound, in the manner to be explained hereinafter, a ribbon of six continuous semipermeable hollow fibers, a top end cap or header 60 and a bottom end cap or header 70. The blood oxygenator may optionally include a heat exchanger 80, which as shown in Fig. 3 may be associated with the bottom header which includes an inlet 74 for the introduction into the oxygenator of blood taken from a patient.

Outer casing 30 has a cylindrical peripheral wall 32 and, prior to assembly of the oxygenator, is open at both its ends. The exterior surface of the outer casing comprises a first annular flange 34 which is spaced a slight distance downwardly from the upper end of the casing and a second flange 36 which is spaced a distance downwardly from first flange 34. The portion of peripheral wall 32 lying between the first and second annular flanges is provided with a series of generally rectangular openings 37. The function of openings 37 will be explained hereinafter.

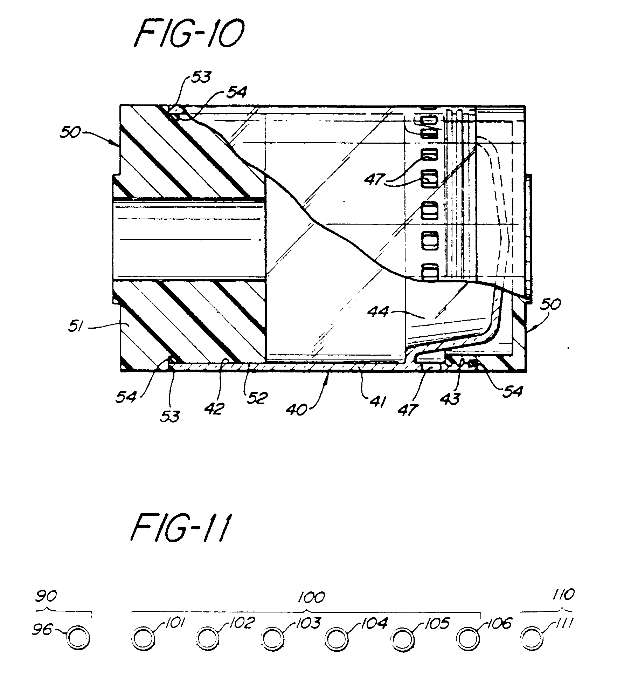

Inner support core 40 comprises a cylindrical peripheral wall 41 which includes an interiorly threaded portion 43 adjacent its bottom end. Support core 40, which prior to assembly of the oxygenator is open at its top end, includes a dish portion 44 which serves to close off its bottom end. Dish portion 44 includes a bottom portion 45 and an angled wall portion 46. As can be seen in Fig. 3 and also in Fig. 10, angled wall portion 46 of dish portion 44 is sealed in fluid tight relationship to the inner surface 42 of peripheral wall 41 at a point located upwardly of threaded portion 43. Peripheral wall 41 is provided with a series of apertures 47 between threaded portion 43 and the point at which angled wall portion 46 is sealed to inner surface 42 of wall 41. The purpose of these apertures will be explained hereinafter.

Top header 60 has an outlet 62 through which, as will be seen hereinafter, blood is removed from the oxygenator after it has been oxygenated. Top header 60 also includes an inlet 64 for the introduction of an oxygenating gas into the lumens of the spirally wound hollow fibers. Top header 60 also includes a recirculation port 65, an arterial blood sampling port 66, and a temperature probe connector 67.

Bottom header 70 has an outlet 72 through which the oxygenating gas is removed from the oxygenator after it has passed through the lumens of the hollow fibers. Bottom header 70 also has a blood inlet 74, an inlet 75 for introducing a heat exchange fluid into heat exchanger 80, and an outlet 76 for withdrawing heat exchange fluid from the heat exchanger.

An annular bundle 85 of spirally wound microporous hollow fibers is located in the space defined by the outer wall of inner support core 40 and the inner wall of outer casing 30. This fiber bundle is embedded in a solidified potting composition at its top and bottom ends. As will be seen hereinafter, the fiber lumens communicate with the outer surface of the upper and lower potted portions 87 and 88, respectively, so that oxygenating gas introduced via gas inlet 64 flows into gas passage 68 in top header 60, then into the open ends of the hollow fibers at the upper surface of the upper potted portion 87, then through the lumens of the hollow fiber, then through the open ends of the hollow fibers at the lower surface of the potted portion 88, then into gas passage 78 in header 70, and finally out of the oxygenator via gas outlet 72.

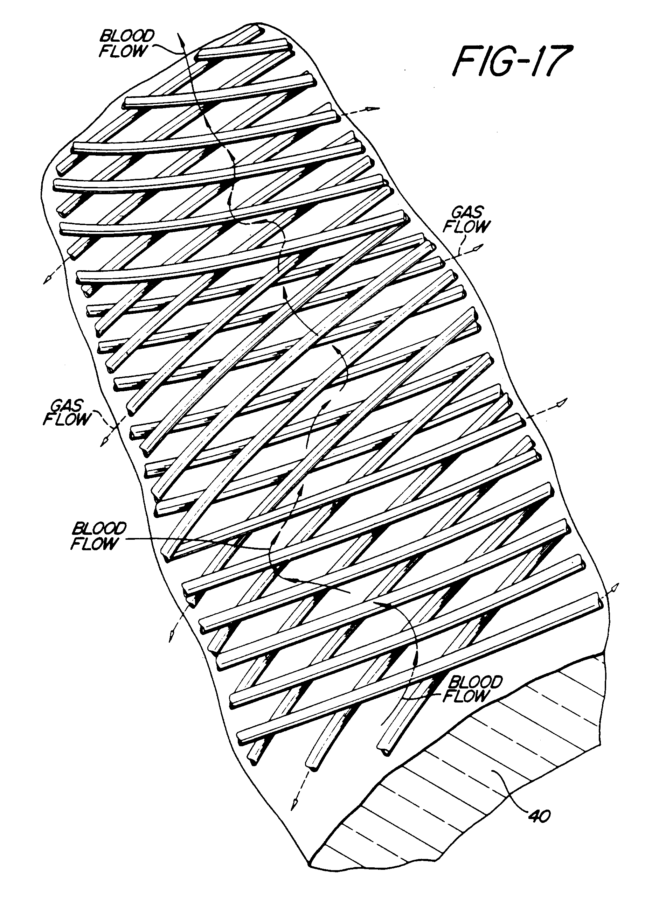

In use, blood to be oxygenated is introduced through blood inlet 74, passes over heat exchanger 80, is directed by the outer surface of dish 44 toward and through openings 47 in support core 40. The blood then flows upwardly, i.e, axially of the oxygenator, over the outer surfaces of the semipermeable hollow fibers. Upon reaching the lower surface of potted portion 87, the blood, now oxygenated, flows through openings 37 in outer casing 30 into arterial blood passage 69 which is defined by the inner surface of skirt portion 63, the upper surface of flange 36, a portion of the lower surface of annular flange 34 and that portion of wall 32 lying between flanges 34 and 36. The blood then exits the oxygenator through blood outlet 62. The arrows in Fig. 17 depict the tortuous path taken by the blood in flowing through the spirally wound fiber bundle. It will be seen that the general flow pattern of the blood is upwardly and axially through the fiber bundle, i.e., in a direction which in general is parallel to the axis of the fiber bundle 85. This flow pattern, in which blood travels over and under the hollow fibers, gives good blood phase mixing which in turn gives excellent gas transfer. The oxygenating gas flows through gas inlet 64 into gas passageway 68, thence into the open ends of the fibers at the outer surface of potted end portion 87, through the fiber lumens, out of the fiber end at the outer surface of potted end portion 88, into gas passage 78, and finally out of the oxygenator through gas outlet 72 in bottom header 70.

The procedure for spirally winding semipermeable hollow fiber on a supporting core will now be described. Referring to Fig. 10, a cylindrical support core 40 is fitted at each of its ends with a cylindrical core extender 50. Core 40 is preferably hollow and has a wall thickness of about 0.125 inch (.32 centimeters). In an illustrative embodiment, the support core is conveniently about 4.5 inches (11.4 centimeters) long and has an outside diameter of about 4 inches (10.2 centimeters). Each core extender 50 comprises a main portion 51 joined to a reduced diameter portion 52 at a shoulder 53. Reduced diameter portion 52 is sized so that its outer surface may be friction fitted with the inner surface 42 of core 40. The outside diameter of core extender 50 corresponds substantially to that of core 40 so that when the extenders are inserted into the ends of the core with shoulders 53 abutting the ends of the support core, there is provided an extended core having a substantially uniform outside diameter. Gaskets 54 are placed around reduced diamter portions 52 near shoulders 53.

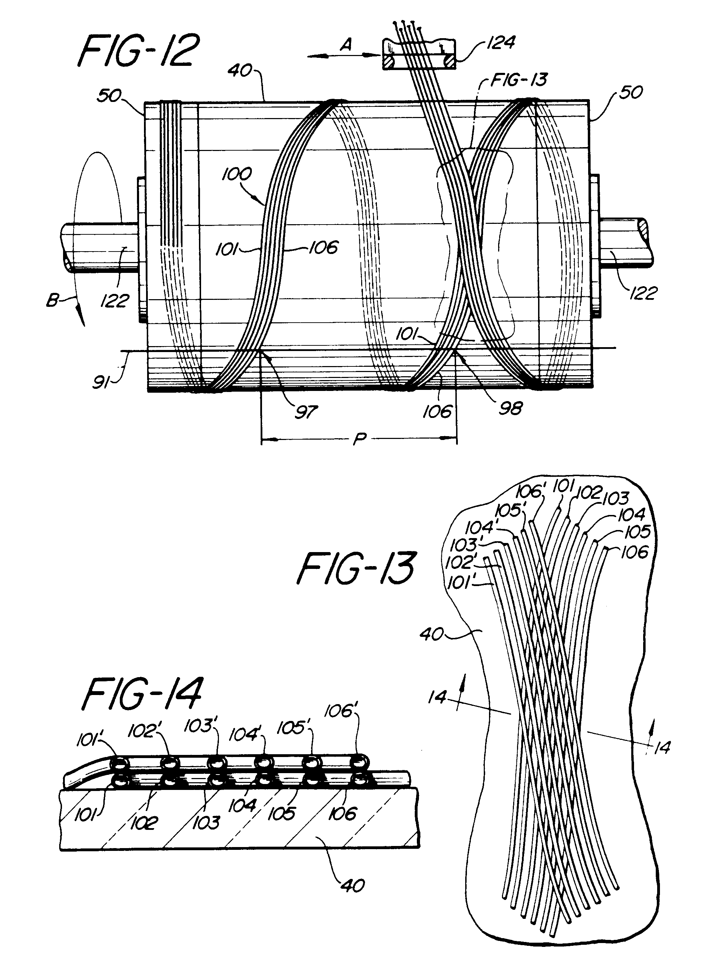

The hollow fiber winding process may be conveniently performed on an apparatus of the type illustrated schematically in Figure 12. The fiber winding apparatus comprises a revolving mounting member 122 and a fiber guide 124 which travels reciprocally, as illustrated by double-headed arrow "A" in Fig. 12, along a line parallel to the axis of rotation of the mounting member. The fiber guide contains a number of upstanding guide pins, not illustred in Fig. 12, through which the fibers are threaded as they enter the guide from a supply container. A winding apparatus sold commercialy by Lesona Corporation under the name Precision Wind Take-Up Machine, Model No. 959 (or equivalent) is suitable for wrapping a continuous hollow fiber (or a number of such hollow fibers) on the extended support core.

The extended core just described is spirally wound with hollow fibers in the following manner. The extended inner support core is mounted on mounting member 122 of the winding apparatus. Guide 124 is then positioned at the left hand side (as viewed in Fig. 12) of the extended core. A ribbon of six continuous semipermeable hollow fibers is taken from a supply container, threaded over an idler roll, under a "dancing" roll, and then through the guide pins of fiber guide 124. Seven such guide pins are used, one continuous hollow fiber being placed between two adjacent pins in order to separate the fibers as they leave the supply continer. The leading end of the fiber ribbon is tied into a small knot which is then affixed, as by taping, to the outer surface of the core extender at the far left end of the extended core. Rotation of mounting member 122 of the winding apparatus is begun in the direction indicated by arrow B in Fig. 12. Guide 124 is geared to mounting member 122 and automatically travels axially of the extended core as mounting member 122 rotates.

Guide 124 travels from the first end (left hand side of Fig. 12) of the extended core to the second end (right hand side of Fig. 12) where, after a brief dwell time, the guide reverses direction and travels back to its starting position. After a brief dwell time at that point, the guide begins its travel cycle anew. This reciprocal travel of guide 124 and the concurrent rotation of mounting member 122 on which the extended support core has been mounted is continued until a fiber bundle of desired diameter has been wound onto the extended core.

Fig. 13 is a greatly enlarged view of the dot-and-dash enclosed portion of Fig. 12 which shows the position of the six fiber ribbon 100 after the fiber guide 124 left its starting position at the left hand side of Fig. 12, traveled to the right hand side of Fig. 12, reversed direction, and traveled leftward to its position illustrated at the top of Fig. 12. In the left-to-right travel of guide 124, the fiber ribbon was wound spirally around the extended support core, and the individual fibers 101-106 in the ribbon were laid down in contact with the outer surfaces of support core 40 and core extenders 50. In the subsequent second traverse (right-to-left in Fig. 12) of guide 124, fiber ribbon 100 continues to be spirally wound onto the extended core. It will be seen that portions of the six fibers (labeled 101', 102', 103', 104', 105', and 106') laid down during the second traverse of the fiber guide contact fibers 101-106 at certain crossover points. Except for these crossover points at which there is fiber-to-fiber contact with fibers laid down during the first traverse of guide 124, the fibers laid down during the second traverse of the fiber guide come into direct contact with the outer surface of the extended core.

Fig. 15A shows the appearance of the fibers on the extended core after the guide 124 has completed 7 traverses in the left-to-right direction, 7 traverses in the right-to-left direction, and is approaching the end of its 8th traverse in the left to right direction. It will be observed that despite the 14+ total traverses of the fiber guide, there still remain certain areas of the support core 40 which have not yet been wound and covered with the fiber ribbon.

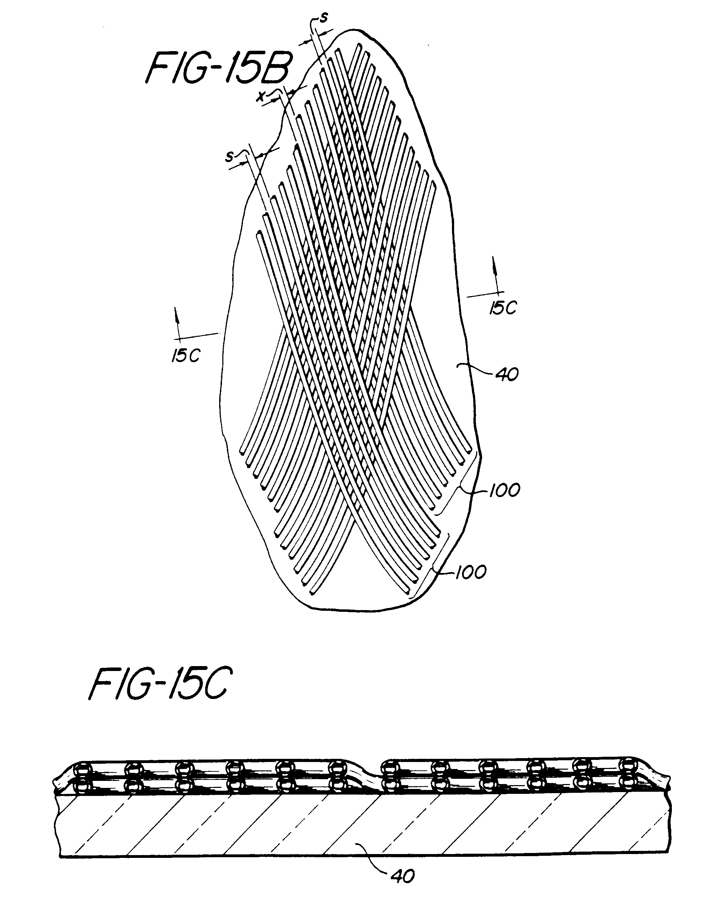

Fig. 15B is a greatly magnified view of the dot-and-dash enclosed portion of Fig. 15A. Reference to Fig. 15B and to Fig. 15C clearly shows that even after nearly 15 total traverses of the fiber guide there is only one thickness of fiber (equal to one fiber diameter) on some portions of the extended support core and at most there are two thicknesses of fiber (equal to two fiber diameters). Areas where fiber coverage is just one fiber diameter thick are illustrated at the upper right hand and upper left hand regions of Fig. 15B. Areas where fiber coverage is just two fiber diameters thick are shown in the lower central region of Fig. 15B and in Fig. 15C.

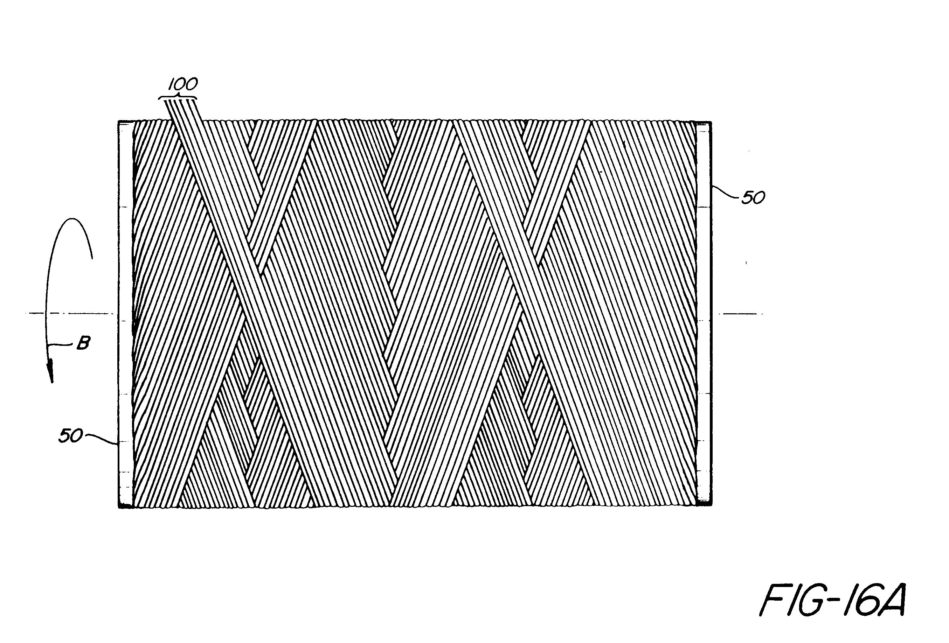

Fig. 16A shows the appearance of the fibers on the extended core at a stage of winding later than that shown in Fig. 15A. In Fig. 16A, the fiber guide has completed 9 traverses in the first direction (left-to-right in Fig. 12) and 8 traverses in the second direction (right-to-left in Fig. 12) and is about to complete its 9th traverse in the second direction.

In the winding procedure being discussed, the extended core is covered, except for the spacing, s, between adjacent fibers and the distance, x, between the sixth fiber of one ribbon and the first fiber of the next adjacent ribbon, when the fiber guide has traveled a total of eighteen traverses, i.e. nine traverses in each direction. Fibers 101-106 of the fiber ribbon laid down during the nineteenth traverse of the fiber guide will be in radial registry with fibers 101-106 laid down during the very first traverse of the fiber guide and fibers 101-106 laid down during the twentieth traverse of the fiber guide will be in radial registry with fibers 101-106 laid down during the second traverse of the fiber guide. Stated more generally, in the particular embodiment under discussion, fibers 101-106 laid down during the $n^{th}$ traverse of the fiber guide will be in registry with fibers 101-106 laid down during the $(n-18)^{th}$, $(n-36)^{th}$, $(n-54)^{th}$ traverse of the fiber guide. For example, fibers 101-106 laid down during the $55^{th}$ traverse will be in radial registry with fibers 101-106 laid down during the $37^{th}$, $19^{th}$ and $1^{st}$ traverses of the fiber guide.

It will also be understood at the completion of the $36^{th}$ traverse of the fiber guide, the thickness of the fiber bundle on the extended core will be equal to four fiber diameters; at the end of the $54^{th}$ traverse, the thickness of the fiber bundle will be equal to six fiber diameters; at the completion of the $72^{nd}$ traverse, the thickness of the fiber bundle will be eight fiber diameters, etc.

It is preferred in carrying out the spiral winding process of the present invention that the spacing, s, between adjacent fibers in a ribbon be the same and that the distance, x, between adjacent fiber ribbons be equal to s.

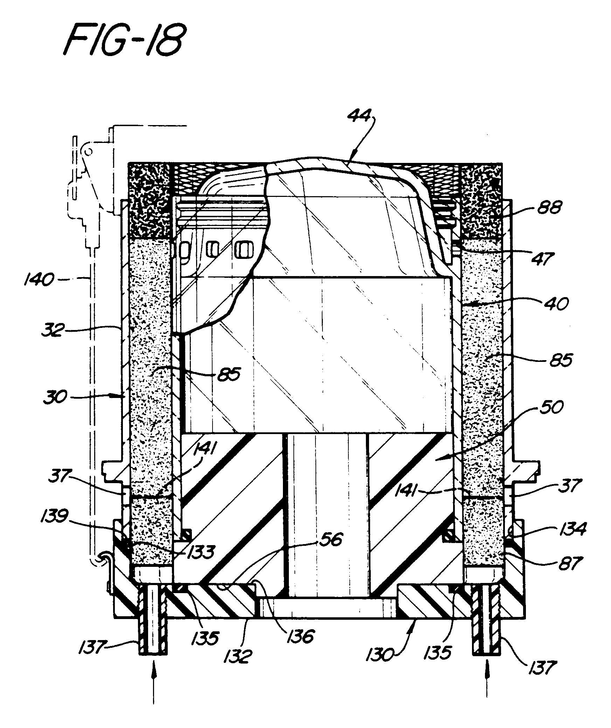

After the desired amount of fiber has been spirally wound onto the extended core in the manner just explained, the extended core with the fiber bundle 85 wound thereon is removed from mounting member 122 of the winding apparatus. The fiber bundle/core combination is then inserted into outer casing 30, the fiber bundle having been sized during the winding procedure so that its outside diameter is about equal to the inside diameter of the outer casing. It is necessary now to apply a liquidform casting resin to the end portions of the fiber bundle, and to allow that resin to harden or cure so as to seal the fibers near the ends of the fiber bundle to each other, to the adjacent outer surface of inner support core 40, and to the adjacent inner surface of outer casing 30. The process, referred to in the art as a "fiber potting process", is carried out with the aid of a potting cap. The bottom portion of Fig. 18 shows potting cap 130 in place over one end of the extended support core/fiber bundle/outer casing combination. Potting cap 130 comprises a circular end portion 132 and a skirt portion 134 depending therefrom. Skirt portion 134 has a shoulder 133 whose width is substantially equal to the thickness of peripheral wall 32 of outer casing 30. When the potting cap is in place, a gasket 139 is placed against shoulder 133 to form a fluid tight seal with the end edge of outer casing 30. Potting cap 130 includes an O-ring 135 placed in a circular groove cut into the inner surface 136 of end portion 132. The groove for the O-ring is located such that when the potting cap is in position, O-ring 135 contacts the periphery of the end face 56 of core extender 50. Potting cap 130 includes two inlets (one inlet would be sufficient) through which the potting resin is applied to the end of the fiber bundle. The potting cap may be secured in place by a friction fit where the inner wall of its skirt portion 134 contacts the outer surface of outer casing 30. Alternatively, a clamping device 140 such as that illustrated in phantom at the left side of Fig. 18 may be used for this purpose.

The liquid potting compound is conveniently applied to the end portions 87, 88 of the fiber bundle 85 as follows. The support core/fiber bundle/outer casing combination, with potting cap 130 in place, is oriented in the direction in which the reader views Fig. 18. A quantity of liquid potting resin is injected, e.g. by using a syringe, into the inlets 137 of potting cap 130. Sufficient resin is injected so that it rises to the level indicated by dashed line 141 and the inlets are closed. Care is taken to make sure the liquid resin rises no higher than the level indicated by line 141 so as to preclude its running through openings 37 in outer casing 30. When the resin level reaches dashed line 141, it is left to cure or harden to its fully solid state.

After the first end of the support core/fiber bundle/outer casing combination has been potted as just described and the potting resin has fully hardened, potting cap 130 is removed and the other end is potted in the same manner. The potting cap is removed from the second end and then both core extenders 50 are removed. This leaves the potted fiber bundle disposed between the outer surface of the inner support core 40 and the inner surface of outer casing 30. The fibers at the end of the fiber bundle are embedded in the cured potting composition. One region of each potted end portion of the fiber bundle is sealed to the outer surface of support core 40 and to the inner surface of outer casing 30. A second region of each potted end portion of the fiber bundle extends beyond the aligned end edges of support core 40 and outer casing 30. Each extending potted end portion is then cut transversely of the longitudinal axis of the support core so that the freshly cut surfaces of the potted portions are flush with the ends of the support core and the outer casing. It will be recognized that the transverse cutting of the potted end portions 87, 88 of the spirally wound fiber bundle provides open ends in the fibers at the outermost cut surface 83 of potted end portion 87 and at the outermost cut surface 84 of potted end portion 88. This provides a continuous path for the flow of a fluid through the lumens of the semipermeable hollow fibers from the cut outer face of one potted end portion of the fiber bundle to the cut outer face of the other potted end portion of the fiber bundle.

After the potting and transverse cutting operations have been completed, gasket 61 is placed around the upper part of first annular flange 34. Top header 60, which has support fins 39 supporting annular flange 36, is then put into position over the open end of support core 40 (see top portion of Fig. 3). Header 60 has a skirt portion 63 whose inner surface, when the header is in position, abuts the outer surface of second annular flange 36. The abutting surfaces may be sealed, e.g. by solvent welding or another suitable method. It is advisable to place a sealing compound 61 in the inverted U-shaped region 29 formed by the lowermost portion of skirt 63 and the projecting tip 28 of annular flange 36.

Bottom header 70 includes an L-shaped flange 92 having a bottom portion 93, an outer side wall 94, and a slanted inner wall 95. A groove is cut into slanted inner side wall 95 to accept a gasket 79. This gasket is put into place and the bottom header 70, which carries outer threads 71, is screwed onto the inner threaded portion 43 of the inner support core. It will be seen that gas passage 78 is defined by the outermost surface of potted end portion 88, gasket 79 and the inner surface of bottom wall 93, side wall 94, and slanted wall 95.

## Claims

1. A spirally wound hollow fibre oxygenator (20), comprising:

    one or more fibre ribbons, each including one or more gas permeable fibres (101-106,101'-106') having continuous lumens therethrough, wound spirally around a core (40) along a substantial portion of the length of the core (40);

    a housing (30) within which the one or more fibres (101-106,101'-106') and the core (40) are disposed such that the one or more fibres (101-106,101'-106') are contained in an annular chamber whose peripheral walls are formed by the housing (30) and the core (40) in such a way that a first plurality of fibre ribbons are wound in one sense, and a second plurality of fibre ribbons are wound in the other sense;

    an annulus of casting resin (87,88) located between and sealed to the core (40) and the housing (30) at each end of the core (40), the end portions of the one or more fibres (101-106,101'-106') being sealed in the resin annuli (87,88) and the ends of the one or more fibres (101-106,101'-106') being open at the outer end surfaces of the resin annuli (87,88), and the resin annuli defining the ends of the chamber containing the one or more fibres (101-106,101'-106');

    end caps (60,70) fitted to the housing (30), one (60) of which end caps has an inlet port (64) for admission of oxygenating gas to the lumens of the fibres and the other (70) of which has an outlet port (72) for exit of oxygenating gas from the lumens of the fibres;

    an inlet (47) for admission of blood to the annular chamber containing the one or more fibres (101-106,101'-106'); and

    an outlet (37) for exit of blood from the annular chamber containing the one or more fibres (101-106,101'-106'); characterised in that:

    the blood inlet (47) and the blood outlet (37) are arranged in the peripheral walls of the annular chamber and spaced axially from one another at opposite ends of the chamber; and

    the fibre ribbon windings occupy substantially all of the annular chamber, thereby constraining the blood to flow axially through the fibre ribbon windings.

2. A hollow fibre oxygenator according to claim 1 which is provided with an integral heat exchanger arranged for raising and lowering the temperature of the blood.

3. A hollow fibre oxygenator according to claim 1 or claim 2 wherein the one or more fibres are polypropylene hollow fibres.

4. A hollow fibre oxygenator according to any one of claims 1 to 3 wherein the oxygenating gas inlet (64) is arranged at the opposite end of the oxygenator to the blood inlet (37), such that the oxygenating gas and blood flows are counter-current.

5. A hollow fibre oxygenator according to any one of claims 1 to 4, wherein the one or more fibres (101-106, 101'-106') include a first fibre ribbon (101-106) wound helically around the core (40) in a first direction from a first end to a second end of the core (40), and a second fibre ribbon (101'-106') wound helically around the core (40) in a second direction opposite to the first direction from the first end of the core (40) to the second end of the core (40).

6. The use of the hollow fibre oxygenator according to any one of the preceding claims to oxygenate the blood of an animal or person extracorporeally, other than in a method for the treatment of the human or animal body by surgery or therapy.

## Revendications

1. Oxygénateur (20) à fibres creuses enroulées en hélice, comprenant :

un ou plusieurs rubans de fibres, dont chacun comprend une ou plusieurs fibres (101-106, 101'-106') perméables aux gaz, comportant un passage continu sur leur longueur, et qui sont enroulés en hélice autour d'un noyau (40) le long d'une partie importante de la longueur du noyau (40) ;

une enveloppe (30) dans laquelle la ou les fibres (101-106, 101'-106') et le noyau (40) sont disposés de manière que la ou les fibres (101-106, 101'-106') soient logées dans une chambre annulaire dont les parois périphériques sont formées par l'enveloppe (30) et le noyau (40) de telle manière que plusieurs premiers rubans de fibres soient enroulés dans un sens et plusieurs seconds rubans de fibres soient enroulés dans l'autre sens ;

un anneau de résine coulée (87, 88) logé entre et lié de manière étanche au noyau (40) et à l'enveloppe (30) à chaque extrémité du noyau (40), les parties extrêmes de la ou des fibres (101-106, 101'-106') étant scellées dans les anneaux de résine (87, 88) et les extrémités de la ou des fibres (101-106, 101'-106') étant ouvertes sur les surfaces extrêmes extérieures des anneaux de résine (87, 88) et les anneaux de résine délimitant les extrémités de la chambre contenant la ou les fibres (101-106, 101'-106') ;

des couvercles d'extrémité (60, 70) ajustés sur l'enveloppe (30), l'un (60) desdits couvercles d'extrémité ayant un accès d'entrée (64) pour l'admission de gaz d'oxygénation dans le passage des fibres et l'autre (70) de ces couvercles comportant un accès de sortie (72) pour l'évacuation du gaz d'oxygénation du passage des fibres ;

une entrée (47) d'admission de sang dans la chambre annulaire contenant la ou les fibres (101-106, 101'-106') ; et

une sortie (37) d'évacuation du sang de la chambre annulaire contenant la ou les fibres (101-106, 101'-106') ;

caractérisé en ce que l'entrée de sang (47) et la sortie de sang (37) sont disposées dans les parois périphériques de la chambre annulaire et sont placées axialement à distance l'une de l'autre aux extrémités opposées de la chambre ; et

les enroulements de rubans de fibres occupent sensiblement la totalité de la chambre annulaire en obligeant ainsi le sang à circuler axialement à travers les enroulements de rubans de fibres.

2. Oxygénateur à fibres creuses selon la revendication 1, dans lequel est incorporé un échangeur de chaleur destiné à élever et abaisser la température du sang.

3. Oxygénateur à fibres creuses selon la revendication 1 ou la revendication 2, dans lequel une ou plusieurs fibres sont des fibres creuses de polypropylène.

4. Oxygénateur à fibres creuses selon l'une quelconque des revendications 1 à 3, dans lequel l'entrée de gaz d'oxygénation (64) est disposée à l'extrémité de l'oxygénateur qui est opposée à celle de l'entrée de sang (37), de sorte que le gaz d'oxygénation et le sang circulent à contre-courant.

5. Oxygénateur à fibres creuses selon l'une quelconque des revendications 1 à 4, dans lequel la ou les fibres (101-106, 101'-106') comprennent un premier ruban de fibres (101-106) enroulé en hélice autour du noyau (40) dans un premier sens d'une première extrémité à une seconde extrémité du noyau (40) et un second ruban de fibres (101'-106') enroulé en hélice autour du noyau (40) dans un second sens opposé au premier sens, de la première extrémité du noyau (40) à la seconde extrémité du noyau (40).

6. Utilisation de l'oxygénateur à fibres creuses selon l'une quelconque des revendications précédentes pour l'oxygénation extra-corporelle du sang d'un animal ou d'une personne autre que suivant un procédé de traitement du corps humain ou animal par chirurgie ou thérapie.

## Patentansprüche

1. Sauerstoff-Zufuhrvorrichtung (20) mit einer spiralförmig gewickelten Hohlfaser, bestehend aus:

einem oder mehreren Faserbändern, von denen jedes eine oder mehrere gasdurchlässige Fasern (101-106,101'-106') mit durchgehendem Lumen aufweist, die spiralförmig um einen Kern (40) entlang eines wesentlichen Teils der Länge des Kerns (40) gewickelt sind;

einem Gehäuse (30), in welchem die eine oder mehrere Fasern (101-106,101'-106') und der Kern (40) derart angeordnet sind, daß die eine oder mehrere Fasern (101-106,101'-106') in einer ringförmigen Kammer enthalten sind, deren Umfangswände von dem Gehäuse (30) und dem Kern (40) derart gebildet sind, daß eine erste Anzahl von Faserbändern in einer Richtung gewickelt ist und eine zweite Anzahl von Faserbändern in der anderen Richtung gewickelt ist;

einem Gußharzring (87,88), der zwischen dem Kern (40) und dem Gehäuse (30) angeordnet und mit diesen an jedem Ende des Kerns (40) verschweißt ist, wobei die Endteile der einen oder mehreren Fasern (101-106,101'-106') in den Kunstharzringen (87,88) versiegelt sind und die Enden der einen oder mehreren Fasern (101-106,101'-106') an den äußeren Endflächen der Kunstharzringe (87,88) offen sind und die Kunstharzringe die Enden der die einen oder mehreren Fasern (101-106,101'-106') enthaltenden Kammer bilden;

Endkappen (60,70), die auf das Gehäuse (30) aufgesetzt sind, wobei eine (60) der Endkappen eine Einlaßöffnung (64) für die Zufuhr des Sauerstoff zuführenden Gases zu den Lumen der Fasern und die andere (70) eine Auslaßöffnung (72) für den Auslaß des Sauerstoff zuführenden Gases aus den Lumen der Fasern aufweist;

einen Einlaß (47) für die Zufuhr von Blut zu der die eine oder mehrere Fasern (101-106,101'-106') enthaltenden Ringkammer; und

einem Auslaß (37) für den Auslaß aus der die eine oder mehrere Fasern (101-106,101'-106') enthaltenden Ringkammer;

dadurch gekennzeichnet, daß

der Bluteinlaß (47) und der Blutauslaß (37) in den Umfangswänden der Ringkammer und in axialem Abstand voneinander an gegenüberliegenden Enden der Kammer angeordnet sind; und

die Faserbandwindungen im wesentlichen die gesamte Ringkammer einnehmen, so daß der Blutstrom axial durch die Faserbandwindungen gezwungen wird.

2. Sauerstoff-Zufuhrvorrichtung nach Anspruch 1, die mit einem integralen Wärmeaustauscher zur Erhöhung und Verringerung der Bluttemperatur versehen ist.

3. Sauerstoff-Zufuhrvorrichtung nach Anspruch 1 oder Anspruch 2, bei der eine oder mehrere Fasern Polypropylen-Hohlfasern sind.

4. Sauerstoff-Zufuhrvorrichtung nach einem der Ansprüche 1 bis 3, bei der der Einlaß (64) für Sauerstoff zuführendes Gas am gegenüberliegenden Ende der Sauerstoff-Zufuhrvorrichtung am Bluteinlaß (37) derart angeordnet ist, daß das Sauerstoff zuführende Gas und Blut im Gegenstrom fließen.

5. Sauerstoff-Zufuhrvorrichtung nach einem der Ansprüche 1 bis 4, bei der die eine oder mehrere Fasern (101-106,101'-106') ein erstes Faserband (101-106) umfassen, das schraubenförmig um den Kern (40) in einer ersten Richtung von einem ersten Ende zu einem zweiten Ende des Kerns (40) gewickelt ist, und ein zweites Faserband (101'-106') schraubenförmig um den Kern (40) in einer zweiten, der ersten Richtung entgegengesetzten Richtung von dem ersten Ende des Kerns (40) zu dem zweiten Ende des Kerns (40) gewickelt ist.

6. Verwendung der Sauerstoff-Zufuhrvorrichtung nach einem der vorhergehenden Ansprüche zum extrakorporealen Anreichern des Blutes eines Tieres oder Menschen mit Sauerstoff im Gegensatz zu einem chirurgischen oder therapeutischen Behandlungsverfahren des menschlichen oder tierischen Körpers.

## FIG-1

# FIG-2

# FIG-3

FIG-4

*FIG-6*

*FIG-5*

FIG-7

FIG-8

FIG-9

## FIG-10

## FIG-11

FIG-12

FIG-13

FIG-14

FIG-15A

FIG-15B

FIG-15C

FIG-16A

BLOOD FLOW

GAS FLOW

GAS FLOW

BLOOD FLOW

BLOOD FLOW

FIG-17

40

FIG-18